# EUROPEAN PATENT SPECIFICATION

(11) **EP 4 338 659 B1**
(45) Date of publication and mention of the grant of the patent: **07.05.2025**
(21) Application number: 22195939.8
(22) Date of filing: 15.09.2022
(51) Int. Cl.: A61B 5/00

(54) **PHOTOACOUSTIC DETECTING DEVICE**
PHOTOAKUSTISCHE DETEKTIONSVORRICHTUNG
DISPOSITIF DE DÉTECTION PHOTOACOUSTIQUE

(43) Date of publication of application: 20.03.2024
(73) Proprietor: Eclypia, 38000 Grenoble (FR)
(72) Inventor: GREFFIER, Damien Patrice, 49440 Challain la Potherie (FR); PALLIER, Cédric, 35470 Bain de Bretagne (FR); BERNET, Florent, 38100 Grenoble (FR)
(74) Representative: Fidal Innovation

(56) References cited:
- WO-A2-2004/107971
- US-A1- 2015 182 167

## Description

### FIELD OF THE INVENTION

The present invention relates to photoacoustic detecting device for measuring a parameter of interest in a medium.

### BACKGROUND OF THE INVENTION

Photoacoustic detection may be used in the field of detecting devices, notably for detecting parameters of interest such as chemical components in a medium. The medium may be an organic tissue, such as the skin of a human.

The photoacoustic detection is based on the irradiation of a medium M to be analysed by a light signal emitted by a light source. Generally, the light source is one or several lasers, in particular a quantum cascade laser (QCL). The light signal is a light beam of a chosen wavelength. The wavelength is chosen regarding the type of parameter of interest to be measured.

The photoacoustic detection is based on the detection of a pressure wave associated with a thermic wave. The thermic wave is generated under the effect of the absorption of the light beam by the medium. Such absorption creates a local heating of chemical components in the medium, where the light beam has been absorbed. The thermic wave propagates in the medium M before propagating outside the medium. More precisely, when the thermic wave comes out of the medium, after its propagation, a pressure wave is generated, which can be detected.

The photoacoustic detection may be made specific to particular chemical components, by adjusting the wavelength and/or the modulation frequency of the light beam. More precisely, the wavelength may be adjusted to match an absorption peak of the component to analyse.

Photoacoustic detection then is a non-invasive way of analysing a medium of interest. Numerous photoacoustic detecting devices have been developed, as for example disclosed in WO2004/107971 and US2015/182167.

Such photoacoustic detecting devices generally include a photoacoustic cell, light source emitting a light beam, a guiding element directing the light beam toward the photoacoustic cell and at least one transducer able to detect a signal generated in the photoacoustic cell by the photothermic effect in the medium in response to the irradiation of the medium by the light beam.

A major drawback of such photoacoustic devices is that they are conceived as one monolithic block which includes the photoacoustic cell, the light source, the guiding element and the transducer. Such a detecting device comprising a monolithic block induces that when one of the elements is broken and need to be replaced or when the parameters of interest need to be measured according to different characteristics induced by the structure of the photoacoustic cell, for example, all the monolithic block needs to be replaced. In other words, the whole detecting device must be replaced.

The present invention thus aims to answer at least partially the above-mentioned problem.

### BRIEF SUMMARY OF THE INVENTION

Thus, the invention relates to a photoacoustic detecting device for measuring a parameter of interest in a medium comprising:
» A housing comprising a baseplate and a cover,
> A light source, configured to emit a light signal
» A photoacoustic cell comprising a contact surface, emerging out of an aperture of the baseplate, said contact surface being intended to be in contact with the medium, wherein the light signal is able to propagate in the photoacoustic cell and pass through the contact surface to reach the medium,
➢ A guiding structure, configured to direct the light signal toward the photoacoustic cell,
➢ A transducer linked to the photoacoustic cell and configured to detect a generated signal, where the generated signal is generated in the photoacoustic cell by a photothermic effect in the medium in response to an irradiation of the medium by the light signal,
➢ A first maintaining element configured to assemble the light source and the guiding element in a building block, said building block being housed in the housing
   Characterised in that
➢ The photoacoustic cell is reversibly attached to the building block by at least one fixing element accessible from an outside of the housing, in that a size of the photoacoustic cell is such that the photoacoustic cell can be removed from the housing by passing through the aperture of the baseplate when needed, and in that
➢ The transducer is reversibly coupled to the building block and placed between said building block and said photoacoustic cell, such that the transducer can be removed from the aperture of the baseplate at the same time or after the photoacoustic cell is being or has been removed from the housing.

The proposed detecting device allows an easy and facilitated dismantling of the photoacoustic cell and/or of the transducer when needed. Indeed, since the fixing elements are accessible from an outside of the housing, there is no need to take the cover out or to open the housing; the photoacoustic cell can be removed directly from the aperture of the baseplate. The other components are really well protected by this particular configuration. Furthermore, since the transducer is reversibly coupled to the building block and placed between the building block and the photoacoustic cell, once the photoacoustic cell has been removed from the housing or at the same time that the photoacoustic cell is being removed from the housing, the transducer can also be removed from the housing without opening the housing.

The dismantling of the photoacoustic cell and/or of the transducer may be needed for cleaning one or both of the photoacoustic cell and transducer, or to replace one or both of the photoacoustic cell and transducer by another photoacoustic cell or transducer. This may be the case when one of these two components is broken, or when a photoacoustic cell or transducer with different properties is needed for the analysis of the parameter of interest.

According to an embodiment, the transducer is attached to a second maintaining element, the second maintaining element comprising a connecting portion in regards with the first maintaining element, and wherein a connecting element is configured to reversibly connect said connecting portion of said second maintaining element to the first maintaining element so as to ensure a mechanical, electronic and/or electrical connection between the transducer and the building block.

Generally, the transducer needs to be linked permanently to the components allowing the analysis of the generated detected signal. Such a link may be wires or brazing of the transducer, for example. In the present application, the connecting element ensures the connection of the transducer to such analysis components while allowing an easy dismantling of the transducer from the detecting device when needed. More particularly, the connecting element allows the mechanical connection of the transducer with the building block such that the transducer is not able to move around in the housing, and/or the electronic connection of the transducer with the building block, the building block carrying components for analysing the generated signal detected by the transducer, and/or the electric connection of the transducer with the building block.

According to an embodiment, the removal of the transducer outside of the housing comprises the removal of the transducer and of the second maintaining element.

When a user needs to remove or clean the transducer, it means that they need to replace the same transducer or a new transducer after. However, it may be complicated to place and secure the transducer inside the housing correctly when the housing is closed. Indeed, if the placement of the transducer is not correct, it may not be able to properly detect a generated signal.

It is an aim of the present application to allow the replacement of the transducer without the need of dismantling the whole detecting device.

Thanks to this particular configuration, when the user needs to replace the transducer, they actually need to replace the assembly made of the transducer and of the second maintaining element. This assembly is much more easier to replace than the transducer thanks to the connecting portion of the second maintaining element that is placed in regards with the connecting element. The user may then be able to detect when the second maintaining element is in the good position, and therefore when the transducer is in the good position.

According to an embodiment, the connecting element is brazed on the first maintaining element.

Since the connecting element is permanently attached in the detecting device, the placement of the assembly made of the transducer and second maintaining element is facilitated for the user.

According to an embodiment, the connecting element is a spring leaf connector.

The fabrication is facilitated since leaf springs are used as components in the electronic field. Furthermore, spring leaf connector are generally inexpensive.

According to an embodiment, the fastening of the photoacoustic cell to the detecting device with the fixing elements is configured to press the second maintaining element against the connecting element.

This configuration thereby ensures the good connection of the transducer to the building block, via the connecting element.

More precisely, the photoacoustic cell comprises an extending portion touching the second maintaining element and surrounding the transducer, said extending portion pressing the second maintaining element against the connecting element.

According to an embodiment, the photoacoustic cell comprises an extending portion reversibly attached to the second maintaining element, said extending portion surrounding the transducer.

Thanks to this configuration, the photoacoustic cell can be removed out of the detecting device without the need to also remove the transducer. This can be particularly advantageous one the user needs to change one of the components. Indeed, the user may simply put a new transducer and replace the old photoacoustic cell (or vice-versa) without replacing both of the transducer and the photoacoustic cell when only one of the two needs to be changed.

According to an embodiment, the extending portion of the photoacoustic cell comprises a stud configured to cooperate with a hole provided on the second maintaining element.

This configuration facilitates the placement of the photoacoustic cell back in the detecting device. Since the tightness between the photoacoustic cell and the transducer needs to be ensured to allow a good photoacoustic detection, the stud and hole allow the user to know exactly when the photoacoustic cell is in the good position inside the detecting device. This is particularly useful when the photoacoustic cell alone is removed from the detecting device and needs to be replaced back.

According to an embodiment, the photoacoustic cell comprises an extending portion permanently attached to the second maintaining element, said extending portion surrounding the transducer.

Thanks to this configuration, the photoacoustic cell and the transducer are removed together from the detecting device. This can be particularly advantageous when the user only needs to clean the two components since only one step is needed to remove the transducer and the photoacoustic cell.

According to an embodiment, the photoacoustic cell is attached to the second maintaining element by an adhesive, such as a double-sided tape.

According to an embodiment, the fixing elements reversibly attaching the photoacoustic cell to the building block are screwing elements, wherein heads of the screwing elements are accessible from outside of the housing.

The skirt may improve the comfort of the user and may hide the fixing elements of the photoacoustic cell.

The present application also relates to a photoacoustic detecting device kit comprising a photoacoustic detecting device according to the application and a plurality of photoacoustic cells presenting different acoustic properties and having same reversible fixing elements such that each photoacoustic cell of the plurality of photoacoustic cells is configured to be interchangeable in the photoacoustic detecting device.

Since the photoacoustic detecting device is dismantlable, providing a kit of different photoacoustic cells allows the user to interchange a cell with another when needed. Advantageously, the cells present different acoustic properties such that different parameter or signal may be measured using different cells.

According to an embodiment, the photoacoustic detecting device kit further comprises a plurality of transducers being of different types and/or presenting different properties, each transducer of the kit of transducers being attached to a maintaining element having the same size and characteristics as the second maintaining element such that all the transducers of the kit of transducers are configured to be interchangeable in the photoacoustic detecting device.

Hence, the user may also change the transducer when different signal needs to be measured, for example.

### BRIEF DESCRIPTION OF THE DRAWINGS

Embodiments of the invention will be described below with reference to the drawings, described briefly below:
[Fig. 1] represents a photoacoustic detecting device worn on the arm of a person P.
[Fig. 2] is a block schema of a photoacoustic detecting device.
[Fig. 3] is a schematic representation of the interior of a photoacoustic detecting device according to an embodiment.
[Fig. 4] is another schematic representation of the interior of a photoacoustic device.
[Fig. 5] is an above view of the second maintaining element.

In the drawings, identical references designate identical or similar objects.

### DETAILED DESCRIPTION OF THE INVENTION

The invention relates to a photoacoustic detecting device 1 for measuring a parameter of interest in a medium M.

In a non-limiting example, the photoacoustic detecting device 1 (or "detecting device 1" in the following description) is intended to be worn by a person P. The medium M may be an organic tissue such as the skin of the person P wearing the detecting device 1.

A parameter of interest may be a chemical component present in the skin of the person P, such as molecules. The parameter of interest may comprise glucose, cholesterol, triglyceride, urea, albumin, and/or alcohol. This list is non exhaustive, and several other parameters of interest may be measured.

The measured parameters may then be analysed to determine a blood concentration of glucose, cholesterol and so on.

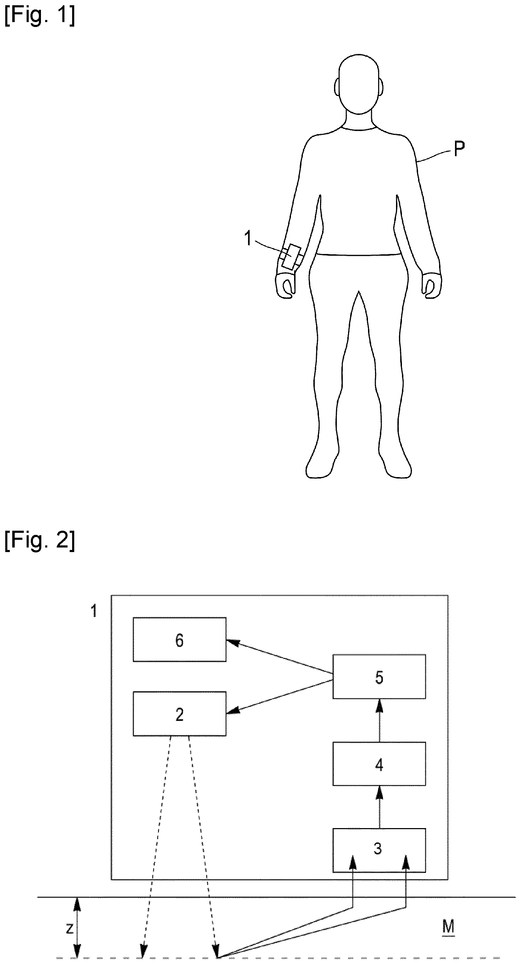

As seen on figure 1, the detecting device 1 may be worn on the arm, or wrist, of a person P. The detecting device 1 may be fixed to the arm of the person P by means of a bracelet.

The detecting device 1 may allow a continuous monitoring of the person P, by repeatedly measuring the parameters of interest of the person P, while it is worn.

Figure 2 is a block diagram of the detecting device 1.

The detecting device 1 comprises:
at least one light source 2, configured to emit a light signal.
a transducer 3 acquiring a signal coming from the medium M,
a signal processing module 4 for analysing the signal detected by the transducer.

In an embodiment, the transducer 3 may be an acoustic transducer detecting an acoustic signal generated in response to the irradiation of the medium M by the light signal

The transducer 3 can be connected to the signal processing module 4 such that the signal processing module 4 receives a signal coming from the transducer 3.

In a non-limiting embodiment, the signal processing module 4 may comprise an analogic-to-numeric converter, converting the signal acquired by the transducer 3 to a numeric signal

In another embodiment, the transducer 3 may directly transmit a numeric signal to the signal processing module 4.

The signal processing module 4 may be implemented in a processor (not shown) which may or not be remote from the detecting device 1.

The detecting device 1 may comprise other components. For example, the detecting device 1 may also comprise an adapting module 5 for adapting irradiation parameters of the light source 2 and a memory 6, for example. These components won't be further described.

In an embodiment, the light source 2 emits a light signal at a chosen wavelength, towards the medium M to be analysed. The chosen wavelength may be chosen according to the parameters of interest to be measured.

More precisely, the wavelength may correspond to the absorption peak of the parameter of interest to be measured. As an example, to detect glucose, the wavelength may be 1034 cm⁻¹, which corresponds to the absorption peak of the glucose.

The light source may be a laser, and more particularly a quantum cascade laser (QCL). In a variant embodiment, the light source 2 may be an electroluminescent diode (led).

In an embodiment, the detecting device 1 may comprise a plurality of light sources 2. In this embodiment, each light source 2 may emit a light signal at different wavelengths. In variant, some light sources 2 emit a light signal at the same wavelength, while other light sources 2 emit a light signal at a different wavelength.

In the following description, the detecting device 1 is described as comprising only one light source 2, while it is understood that the same specification applies for a plurality of light sources 2.

The light signal emitted by the light source 2 propagates to the medium M and through it. This phenomenon is represented by the dotted arrows on figure 2. The light signal is absorbed by the constituents of the medium M under a depth z depending on the chosen wavelength of the light signal, the frequency of the light signal and of the composition of the medium M.

The absorption of the light signal energy causes a local heating of the medium M. Therefore, a thermic signal propagates in the medium M (phenomenon illustrated by the full arrows on figure 2), in particular towards the surface of the medium M. Moreover, the thermic wave may create, outside the medium M, a pressure wave that propagates outside the medium M. Such a pressure wave may be detected by the acoustic transducer 3.

In an embodiment, the thermic wave may also be detected by means of a thermic transducer, such as a thermometer, not shown in the figures.

The detecting device 1 may comprise an acoustic and/or a thermic transducer 3. Other types of transducers 3 may be used.

On the figures, two transducers 3 are shown, while only one or more than two transducers 3 may be used.

In the following description, the detecting device 1 is described as comprising only one transducer 3. It will be understood that what is described for one transducer applies to several transducers 3.

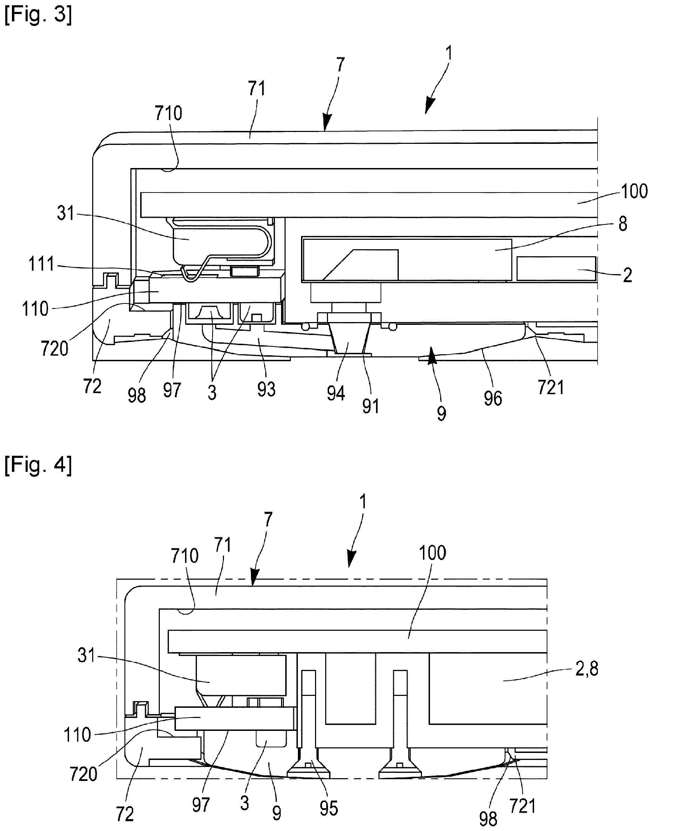

Figure 3 schematically illustrates a detecting device 1 according to an embodiment.

The detecting device 1 may comprise a housing 7, formed by a cover 71 and a baseplate 72. The housing 7 comprises an interior delimited by an interior surface 710 of the cover 71 and an interior surface 720 of the baseplate 72.

The interior of the housing 7 houses a light source 2, which, as described above, may be a QCL.

A guiding element 8 is also provided. The guiding element 8 is configured to direct the light signal emitted by the light source 2 towards the medium M to be analysed.

In the embodiment in which the detecting device 1 comprises a plurality of light sources 2, the guiding element 8 may also be configured to collimate each light signal emitted by each light source 2 into a single light signal directed towards the medium M to be analysed.

More particularly, the guiding element 8 is configured to direct the light signal emitted by the light source 2 through a photoacoustic cell 9 (or "cell 9" in the following description), placed between the medium M to be analysed and the light source 2.

The cell 9 extends from the guiding element 8 to a hollow contact surface 91 of said cell 9 through which the medium M is accessible. The cell 9 comprises a cavity 92 and a channel 93. The light signal emitted by the light source 92 propagates in the photoacoustic cell 9 by first entering the cavity and then passing through the hollow contact surface 91.

The hollow surface contact 91 may generally present the shape of a ring. The hollow contact surface 91 may have the shape of a circle and the hollow may be positioned on the centre of the circle and may also present a general circular shape. Other configurations are possible.

The hollow part of the hollow contact surface 91 may be closed by a window of transparent material such as silicon, allowing the light signal to traverse it.

The hollow surface contact 91 may be intended to be placed against the medium M to be analysed, and more precisely may be placed in direct contact with the medium M to be analysed.

Then, when the detecting device 1 is worn by a person P, as represented on figure 1, the hollow contact surface 91 is directly placed against the skin of the person P.

To this end, the base plate 72 comprises an aperture 721 from which the hollow contact surface 91 emerges. Thus, the hollow contact surface 91 of the cell 9 is positioned outside the interior of the housing 7 and can be placed in direct contact with the medium M to analyse.

Regarding the photoacoustic detection, a thermic wave is generated under the effect of the absorption by the medium of the light beam emitted by the light source. Such absorption creates a local heating of chemical components in the medium, where the light beam has been absorbed. The thermic wave propagates in the medium M before propagating outside the medium. When the thermic wave comes out of the medium, after its propagation, a pressure wave is generated, which propagates in the cell 9 where it can be detected. The pressure wave, i.e. an acoustic wave, is then induced by the photothermic effect.

The transducer 3 is located in the interior of the housing 7 and is preferably linked to the cell 9 by means of the channel 93 comprising an outlet facing the transducer 3, such that the transducer 3 can detect and measure the generated pressure wave. More precisely, the generated pressure wave propagates from the hollow contact surface 91, then inside the channel 93 to finally reach the transducer 3 through the outlet of the channel 93.

In the embodiment where the detecting device 1 comprises more than one transducer 3, the channel 93 may comprise as many outlets as there are transducers 3.

As sometimes it may happen that one of the elements of the detecting device 1 needs to be replaced for a reason, the detecting device according to the present application is conceived to be modular, i.e. with elements being able to be changed or replaced without the need to replace the whole detecting device 1. Such elements may in particular be the photoacoustic cell 9 and/or the transducer 3. A reason of such need to replace it is that the element may be broken, that the element needs to be cleaned or that a parameter of interest may be analysed according to different characteristics not allowed by the used photoacoustic cell 9 and/or transducer.

To solve this technical problem, the detecting device is provided with a first maintaining element 100. The first maintaining element 100 may be a metallic plate or a printed cardboard (PCB). The first maintaining element 100 is used to maintain the light source 2 and the guiding element 8 together, among other electronic components not described herein.

In an example, the first maintaining element 100 maintains the light source 2 and the guiding element 8 in a non-removable way, meaning that the light source and the guiding element cannot or cannot easily be detached from the maintaining element.

For example, the light source 2 and the guiding element 8 may be attached to the maintaining element by screwing elements, not accessible to a user. The screwing element may be accessible only by completely dismantling the detecting device 1.

Hence, the first maintaining element 100, the light source 2 and the guiding element 8 form one single building block. The single building block is considered cinematically as a rigid body in normal use.

The single building block may also comprise the analogic-to-numeric converter, the signal processing module, the adapting module 5 and/or the memory 6.

In the following description, it is considered that the first maintaining element is a first PCB 100, while other applications and embodiments enter within the scope of the present application.

The first PCB 100 also ensures the electronic and/or electric connections between the components of the building block. In other words, the first PCB ensures the electronic, the electric and the mechanical connections between the components of the building block.

Regarding the attachment of the photoacoustic cell 9 in the detecting device 1, it can be more precisely seen on figure 4.

As stated above, the cell 9 comprises a hollow contact surface 91 which emerges out of an aperture 721 of the baseplate 72.

The hollow contact surface 91, viewed from the outside of the housing 7 generally presents a ring-shape. As viewed from profile on figures 3 or 4, the hollow contact surface 91 presents a domed-shape or a conical-shape. Such shape helps to keep constant contact between the medium to analyse and the hollow contact surface 91.

Other shapes may also be used and enter within the scope of the present application.

To attach the photoacoustic cell 9 to the rest of the detecting device 1, fixing elements 95 may be used. Such fixing elements may be used to removably attach the photoacoustic cell to the guiding element 8. The fixing elements 95 may be screwing elements, the heads of the screwing elements 95 being accessible from the outside of the housing 7. Alternatively, the fixing elements may be clipping elements.

Hence, if needed, the photoacoustic cell 9 may be easily removed from the detecting device 1 by unfastening the fixing elements 95, i.e. unscrewing the screwing elements or unclipping the clipping elements, without the need of dismantling the whole detecting device 1. To permit such dismantling, the photoacoustic cell 9 and the aperture of the baseplate 72 are consequently sized.

A skirt 96 may be provided on top of the hollow contact surface 91, without covering the hollow of said hollow contact surface 91. The skirt 96 may extend beyond the hollow contact surface 91, onto the external surface of the baseplate 72. The skirt 96 may be in a soft and/or slightly elastic material such as rubber or silicone.

An advantage of this embodiment is that the material used for the skirt and the glueing between the skirt 96 and the cell 9 allows to ensure the tightness, i.e. water tightness but also dust tightness, of the cell 9 relative to the environment.

As an alternative, a sealing element 98 may be provided between the skirt 96 and the cell 9 to ensure the tightness. For example, the sealing element 98 may be overmolded on the hollow contact surface of the cell 9 or on the perimeter of the skirt or the sealing element 98 may be inserted between the hollow contact surface of the cell 9 and the skirt 96 and glued on the hollow contact surface of the cell 9 or on the perimeter of the skirt 96.

The skirt 96 may hide the heads of the screwing elements 95. Hence, to remove the photoacoustic cell 9 when needed, one may use any suitable product able to detach the skirt 96 from the hollow contact surface 91. Such product may be a solvent or alcohol.

In another embodiment, the skirt 96 may be a clipsable skirt 96, clipsable on a pin (not shown) provided on the hollow contact surface 91 of the cell 9. The skirt 96 may be in a soft and elastic material such as plastic or silicone. In this embodiment, one may unclip first the skirt 96 from the cell 9 to access the screwing elements 95.

To ensure the tightness of the cell 9 relative to the environment, i.e. water and/or dust tightness, a sealing element 98 may be provided between the skirt 96 and the cell 9 to ensure the tightness. For example, the sealing element 98 may be overmolded on the hollow contact surface of the cell 9 or on the perimeter of the skirt or the sealing element 98 may be inserted between the hollow contact surface of the cell 9 and the skirt 96 and glued on the hollow contact surface of the cell 9 or on the perimeter of the skirt 96.

While the use of the skirt 96 is optional, it may improve the contact between the hollow surface contact 91 and the medium to analyse, it may improve the comfort of the user wearing the detecting device 1 and it may improve the holding between the photoacoustic cell 9 and the housing.

Furthermore, it can be seen from figure 3 that the cell 9 comprising the hollow contact surface 91, the cavity 92 and the channel 93 is formed as a whole, i.e., as a single piece. The cell 9 may be made in one material, for example in silicon or resin, or the cell may be made of two or more materials, for example silicon and resin.

To have a photoacoustic cell 9 as one single piece allows to reduce the cost of fabrication while allowing an easy and fast assembly of the cell 9 to the detecting device 1.

To enable the detection of the generated signal by the transducer 3, a second maintaining element 110 is provided in the detecting device 1.

The second maintaining element 110 may be a metallic plate or a printed cardboard (PCB). In the following description, it is considered that the second maintaining element is a second PCB 110, while other applications and embodiments enter within the scope of the present application.

As can be seen on figure 3, the second PCB 110 comprises a first face directed toward the first PCB 100 and a second face direct toward the baseplate 72.

In an embodiment, the transducer 3 is maintained to the second face of the second PCB 110 by brazing. In this embodiment, the transducer 3 is non removably attached to the second PCB 110.

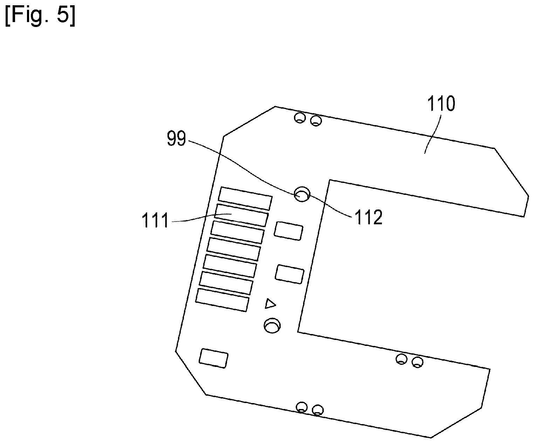

Hence, when the user needs to remove the transducer 3 out of the detecting device 1, the transducer 3 and the second maintaining element are removed together attached. Since the transducer 3 and the second maintaining element are removed from the aperture of the baseplate 72, the second maintaining element 110 may be sized to fit through the aperture 721 of the baseplate. In an example visible on figure 5, the maintaining element 110 can take the general form of a U.

As the transducer 3 detects the generated signal, the information contained in the generated signal may be sent to components participating in the analysis of said detected generated signal. Said components may be the analogic-to-numeric converter or the signal processing module 4. In a preferred embodiment, the first PCB 100 carries the analogic-to-numeric converter or the signal processing module 4 or at least carries the means necessary to send the information detected by the transducer to said analogic-to-numeric converter or said signal processing module 4. Hence, a bridge may be implemented between the first PCB 100 and the transducer 3.

With respect to the need of having a modulable detecting device 1 where the transducer 3 may be decoupled from the rest of the detecting device 1, the bridge may be created by a connecting element. The connecting element may take the form of a leaf spring connector 31. In the following description, it is considered that the connecting element is a leaf spring connector, while other types of connectors having the same function as the spring leaf connector may be used.

Advantageously, the second PCB 110 comprises connecting portions 111. As can be seen on figure 3, the leaf spring connector 31 may link the connecting portions 111 of the second PCB 110 to the first PCB 100 on which the leaf spring connector 31 is brazed.

In an embodiment, the leaf spring connector 31 is brazed on the first PCB 100 such that the leaf spring connector 31 cannot be removed from inside the detecting device 1 without dismantling the detecting device 1.

Advantageously, the distance between the first and second PCBs 100, 110 is such that the leaf spring connector 31 placed between them is in a slight compression state.

Advantageously, the compression state is obtained by attaching the fixing element of the cell 9 to the detecting device, i.e. by screwing the screwing elements 95 or by clipping the clipping element.

Hence, the leaf spring connector 31 as well as the connecting portions 111 together allow an electronic, mechanical and/or electrical connection between the transducer 3 and the rest of the electronics, and more preferably, they allow an electronic, mechanical and electrical connection between the transducer 3 and the rest of the electronics.

Such configuration allows a very easy fabrication since leaf springs are commonly used as components in the electronic field. It also reduces the cost of fabrication. Furthermore, the elasticity of the leaf spring connector 31 in its compression state ensures a permanent connection between the connecting portions 111 of the first and second PCBs 100, 110 when the detecting device 1 is used. Finally, the use of the leaf spring connector 31 provided a mechanical, electronic and/or electrical connection without any fixing element.

In an embodiment, each PCB may comprise several connecting portions 111 and several leaf springs connectors 31 may be used.

Furthermore, the second PCB 110 may be used to allow the mechanical connection between the cell 9 and the transducer. In an embodiment, the cell 9 may present extending portions 97 in contact with the second face of the second PCB 110 surrounding the transducer 3. To perform the mechanical connection between the cell 9 and the second PCB 110, an adhesive may be applied between the extending portions 97 of the cell and the second face of the second PCB 110. Such adhesive may be a non-permanent adhesive, such that the cell 9 may be detached from the second PCB 110 when needed. As an example, such adhesive may be a double-sided tape.

According to the above-mentioned embodiments, when there is a need to access the photoacoustic cell 9 and/or the transducer 3, the user may simply remove the photoacoustic cell 9 from the detecting device and replace it with another. To do so, the user may first detach the skirt 96 from the baseplate 72 by using a solvent or unclip the skirt 96, if appropriate. Then, unscrew the screwing elements 95 and remove the photoacoustic cell 9 from the inside of the housing through the aperture 721 of the baseplate 72. Advantageously, by gently pulling the photoacoustic cell 9 through the aperture, the extending portions 97 of the cell 9 will get detached from the second PCB 110 due to the type of adhesive used to maintain the cell 9 to said second PCB 110.

If the transducer 3 needs to be changed, the same operation is made regarding the photoacoustic cell 9 and then, the transducer 3 brazed to the second PCB 110 and the second PCB 110 can be extracted from the housing 7 through the aperture 721 of the baseplate 72.

To replace the transducer 3 and the photoacoustic cell 9, the user may insert a new or the same transducer in the detecting device 1 through the aperture 721 of the baseplate. Advantageously, if a new transducer 3 is placed, the transducer 3 is already brazed on a PCB corresponding to the second PCB 110. The second PCB 110 to which the transducer 3 is brazed is inserted such that the connecting portions of the second PCB 110 face the leaf spring connector 31.

Then, the photoacoustic cell 9 or a new photoacoustic cell is placed inside the detective device 1 through the aperture 721.

To facilitate the installation of the photoacoustic cell 9 inside the detecting device 1, a stud 99 may be provided on the extending portions 97 of the cell 9, the stud 99 being cooperating with corresponding holes 112 on the second PCB 110. This is visible on figure 5. Hence, the user may be able to know when the photoacoustic cell 9 is in place. Furthermore, the stud 99 and the hole 112 are used to align the transducer 3 and the channel 94 of the cell 9, to allow having a tightness between the channel 94 of the cell 9 and the transducer 3. In other words, the stud 99 and hole 112 help to avoid any leak, due to a poor tightness or poor alignment between the cell 9 and the transducer 3, in the detection of the signal.

In another embodiment, the adhesive used to maintain the cell 9 to the second PCB 110 does not allow to separate said cell 9 and said second PCB 110 by simply pulling the cell 9 out of the housing. Hence, when the cell 9 is removed from the housing 7, the transducer 3 and the second PCB 110 also come out of the housing together with the cell 9.

In an embodiment, outside of the housing 7, the user may be able to detach the cell 9 from the second PCB 110 by pulling it. Then, the transducer and/or the photoacoustic cell 9 or a new transducer 3 and/or a new photoacoustic cell 9 can be replaced as described above.

In another embodiment, the cell 9 cannot be detached from the second PCB 110 due to the type of adhesive used. Hence, after having removed the assembly formed by the transducer, the second PCB and the cell, the user may replace the same assembly or new assembly comprising a transducer brazed on a second PCB and a photoacoustic cell glued to the second PCB.

In all the embodiments described above, after having replaced the elements inside the detecting device, the user may then screw back the screwing elements 95 and, if applicable, place the skirt 96 on top of the hollow contact surface of the cell 9.

As stated above, it may exist a need to use photoacoustic cells 9 and/or transducers having different characteristics, such that different parameters of interest may be analysed.

To this aim, the detecting device 1 may be provided with a kit of different photoacoustic cells 9 and/or different transducers 3.

Advantageously, the transducers 3 of the kit are already brazed to a PCB.

Advantageously, the kit comprises a plurality of assembly comprising a photoacoustic cell glued to a PCB and a transducer brazed to said PCB. Each assembly may comprise different characteristics allowing different analysis and detection of parameters of interest.

The transducers included in the kit may be of different types, for example thermic, acoustic, etc. The photoacoustic cells 9 included in the kit may also have different features. In a non-limitative embodiment, some cells 9 of the kit may comprise a hollow surface contact 91 or may comprise a window recovering the hollow surface contact 91. Other features can be implemented.

While exemplary embodiment of the invention has been described with reference to two main embodiments, it is intended that the invention is not limited to the particular embodiment disclosed as the best mode contemplated for carrying out this invention, but that the invention will include all embodiments falling within the scope of the appended claims. Moreover, unless specifically stated any use of the terms first, second, etc. do not denote any order or importance, but rather the terms first, second, etc. are used to distinguish one element from another.

## Claims

1. Photoacoustic detecting device (1) for measuring a parameter of interest in a medium comprising:
➢ A housing (7) comprising a baseplate (72) and a cover (71),
➢ A light source (2), configured to emit a light signal
➢ A photoacoustic cell (9) comprising a hollow contact surface (91), emerging out of an aperture (721) of the baseplate (72), said hollow contact surface (91) being intended to be in contact with the medium, wherein the light signal is able to propagate in the photoacoustic cell (9) and pass through the hollow contact surface (91) to reach the medium,
➢ A guiding element (8), configured to direct the light signal toward the photoacoustic cell (9),
➢ A transducer (3) linked to the photoacoustic cell (9) and configured to detect a generated signal, where the generated signal is generated in the photoacoustic cell (9) by a photothermic effect in the medium in response to an irradiation of the medium by the light signal,
➢ A first maintaining element (100) configured to assemble the light source (2) and the guiding element (8) in a building block, said building block being housed in the housing (7)
**Characterised in that**
➢ The photoacoustic cell (9) is reversibly attached to the building block by at least one fixing element (95) accessible from an outside of the housing (7), **in that** a size of the photoacoustic cell (9) is such that the photoacoustic cell (9) can be removed from the housing (7) by passing through the aperture (721) of the baseplate (72) when needed, and **in that**
➢ The transducer (3) is reversibly coupled to the building block and placed between said building block and said photoacoustic cell (9), such that the transducer (3) can be removed from the aperture (721) of the baseplate (72) at the same time or after the photoacoustic cell (9) is being or has been removed from the housing (7).

2. Photoacoustic detecting device (1) according to claim 1, wherein the transducer (3) is attached to a second maintaining element (110), the second maintaining element (110) comprising a connecting portion (111) in regards with the first maintaining element (100), and wherein a connecting element is configured to reversibly connect said connecting portion (111) of said second maintaining element (110) to the first maintaining element (100) so as to ensure a mechanical, electronic and/or electrical connection between the transducer (3) and the building block.

3. Photoacoustic detecting device (1) according to claim 2, wherein the removal of the transducer (3) outside of the housing (7) comprises the removal of the transducer (3) and of the second maintaining element (110).

4. Photoacoustic detecting device (1) according to claims 2 or 3, wherein the connecting element is brazed on the first maintaining element (100).

5. Photoacoustic detecting device (1) according to any of claims 2 to 4, wherein the connecting element is a spring leaf connector (31).

6. Photoacoustic detecting device (1) according to any of claims 2 to 5, wherein the fastening of the photoacoustic cell (9) to the detecting device (1) with the fixing element (95) is configured to press the second maintaining element (110) against the connecting element.

7. Photoacoustic detecting device (1) according to any of claims 2 to 6, wherein the photoacoustic cell (9) comprises an extending portion (97) reversibly attached to the second maintaining element (110), said extending portion (97) surrounding the transducer (3).

8. Photoacoustic detecting device (1) according to claim 7, wherein the extending portion (97) of the photoacoustic cell (9) comprises a stud (97) configured to cooperate with a hole (112) provided on the second maintaining element (110).

9. Photoacoustic detecting device (1) according to any of claims 2 to 6, wherein the photoacoustic cell (9) comprises an extending portion (97) permanently attached to the second maintaining element (110), said extending portion surrounding the transducer (3).

10. Photoacoustic detecting device (1) according to claims 2 to 9, wherein the photoacoustic cell (9) is attached to the second maintaining element (110) by an adhesive, such as a double-sided tape.

11. Photoacoustic detecting device (1) according to any of claims 1 to 10, wherein the fixing element (95) reversibly attaching the photoacoustic cell (9) to the building block are screwing elements, wherein heads of the screwing elements are accessible from outside of the housing (7).

12. Photoacoustic detecting device (1) according to any of claims 1 to 11, further comprising a skirt (96) provided on the surface of the hollow contact surface (91) of the photoacoustic cell (9), said skirt (96) being reversibly attached to the detecting device (1).

13. Photoacoustic detecting device kit comprising:
➢ a photoacoustic detecting device (1) according to any of claims 1 to 12,
➢ a plurality of photoacoustic cells (9) presenting different acoustic properties and having same reversible fixing element (95) such that each photoacoustic cell (9) of the plurality of photoacoustic cells (9) is configured to be interchangeable in the photoacoustic detecting device (1).

14. Photoacoustic detecting device kit according to claim 13, further comprising a plurality of transducers (3) being of different types and/or presenting different properties, each transducer (3) of the kit of transducers (3) being attached to a maintaining element having the same size and characteristics as the second maintaining element (110) such that each transducer (3) of the plurality of transducers (3) is configured to be interchangeable in the photoacoustic detecting device (1).

## Patentansprüche

1. photoakustisches Erfassungsgerät (1) zum Messen eines interessierenden Parameters in einem Medium, umfassend:
> ein Gehäuse (7), umfassend eine Grundplatte (72) und eine Abdeckung (71),
> eine Lichtquelle (2), die so konfiguriert ist, dass sie ein Lichtsignal aussendet
> eine photoakustische Zelle (9) mit einer hohlen Kontaktfläche (91), die aus einer Öffnung (721) der Grundplatte (72) herausragt, wobei die hohle Kontaktfläche (91) dazu bestimmt ist, mit dem Medium in Kontakt zu stehen, wobei sich das Lichtsignal in der photoakustischen Zelle (9) ausbreiten und durch die hohle Kontaktfläche (91) hindurchtreten kann, um das Medium zu erreichen,
> ein Führungselement (8), das so konfiguriert ist, dass es das Lichtsignal in Richtung der photoakustischen Zelle (9) leitet,
> ein Wandler (3), der mit der photoakustischen Zelle (9) verbunden und so konfiguriert ist, dass er ein erzeugtes Signal erfasst, wobei das erzeugte Signal in der photoakustischen Zelle (9) durch einen photothermischen Effekt in dem Medium als Reaktion auf eine Bestrahlung des Mediums durch das Lichtsignal erzeugt wird,
> Ein erstes Halteelement (100), das so konfiguriert ist, dass es die Lichtquelle (2) und das Führungselement (8) in einem Baustein zusammenfügt, wobei der Baustein in dem Gehäuse (7) untergebracht ist,
**dadurch gekennzeichnet, dass**
die photoakustische Zelle (9) durch mindestens ein von außerhalb des Gehäuses (7) zugängliches Befestigungselement (95) reversibel an dem Baustein befestigt ist, dass die photoakustische Zelle (9) so bemessen ist, dass sie bei Bedarf durch die Öffnung (721) der Grundplatte (72) aus dem Gehäuse (7) entfernt werden kann, und dass der Wandler (3) reversibel mit dem Baustein gekoppelt und zwischen dem Baustein und der photoakustischen Zelle (9) angeordnet ist, so dass der Wandler (3) gleichzeitig mit der photoakustischen Zelle (9) oder nach deren Entfernen aus dem Gehäuse (7) aus der Öffnung (721) der Grundplatte (72) entfernt werden kann.

2. Photoakustisches Erfassungsgerät (1) nach Anspruch 1, wobei der Wandler (3) an einem zweiten Halteelement (110) angebracht ist, das zweite Halteelement (110) einen Verbindungsabschnitt (111) in Bezug auf das erste Halteelement (100) umfasst, und wobei ein Verbindungselement so konfiguriert ist, dass es den Verbindungsabschnitt (111) des zweiten Halteelements (110) reversibel mit dem ersten Halteelement (100) verbindet, um eine mechanische, elektronische und/oder elektrische Verbindung zwischen dem Wandler (3) und dem Baustein sicherzustellen.

3. Photoakustische Detektionsvorrichtung (1) nach Anspruch 2, wobei das Entfernen des Wandlers (3) aus dem Gehäuse (7) das Entfernen des Wandlers (3) und des zweiten Halteelements (110) umfasst.

4. Photoakustische Detektionsvorrichtung (1) nach Anspruch 2 oder 3, wobei das Verbindungselement an dem ersten Halteelement (100) angelötet ist.

5. Photoakustische Detektionsvorrichtung (1) nach einem der Ansprüche 2 bis 4, wobei das Verbindungselement ein Federblattverbinder (31) ist.

6. Photoakustische Detektionsvorrichtung (1) nach einem der Ansprüche 2 bis 5, wobei die Befestigung der photoakustischen Zelle (9) an der Detektionsvorrichtung (1) mit dem Fixierelement (95) so gestaltet ist, dass das zweite Halteelement (110) gegen das Verbindungselement gedrückt wird.

7. Photoakustische Detektionsvorrichtung (1) gemäß einem der Ansprüche 2 bis 6, wobei die photoakustische Zelle (9) einen Fortsatz (97) umfasst, der reversibel an dem zweiten Halteelement (110) angebracht ist, wobei der Fortsatz (97) den Wandler (3) umgibt.

8. Photoakustische Detektionsvorrichtung (1) nach Anspruch 7, wobei der sich erstreckende Abschnitt (97) der photoakustischen Zelle (9) einen Bolzen (97) umfasst, der so konfiguriert ist, dass er mit einem Loch (112) zusammenwirkt, das an dem zweiten Halteelement (110) vorgesehen ist.

9. Photoakustische Detektionsvorrichtung (1) gemäß einem der Ansprüche 2 bis 6, wobei die photoakustische Zelle (9) einen Fortsatz (97) umfasst, der permanent an dem zweiten Halteelement (110) angebracht ist, wobei der Fortsatz den Wandler (3) umgibt.

10. Photoakustische Detektionsvorrichtung (1) gemäß den Ansprüchen 2 bis 9, wobei die photoakustische Zelle (9) an dem zweiten Halteelement (110) durch einen Klebstoff, wie ein doppelseitiges Klebeband, befestigt ist.

11. Photoakustische Detektionsvorrichtung (1) gemäß einem der Ansprüche 1 bis 10, wobei das Befestigungselement (95), das die photoakustische Zelle (9) reversibel an dem Baustein befestigt, Schraubelemente sind, wobei die Köpfe der Schraubelemente von außerhalb des Gehäuses (7) zugänglich sind.

12. Photoakustische Detektionsvorrichtung (1) gemäß einem der Ansprüche 1 bis 11, die ferner eine Schürze (96) umfasst, die auf der Oberfläche der hohlen Kontaktfläche (91) der photoakustischen Zelle (9) vorgesehen ist, wobei die Schürze (96) reversibel an der Detektionsvorrichtung (1) angebracht ist.

13. Photoakustischer Detektionsvorrichtungsbausatz, umfassend:
Ø eine photoakustische Detektionsvorrichtung (1) gemäß einem der Ansprüche 1 bis 12, Ø eine Vielzahl von photoakustischen Zellen (9), die unterschiedliche akustische Eigenschaften aufweisen und dasselbe reversible Befestigungselement (95) besitzen, so dass jede photoakustische Zelle (9) der Vielzahl von photoakustischen Zellen (9) so konfiguriert ist, dass sie in der photoakustischen Detektionsvorrichtung (1) austauschbar ist.

14. Photoakustische Erfassungsvorrichtung gemäß Anspruch 13, die ferner eine Vielzahl von Wandlern (3) umfasst, die von unterschiedlicher Art sind und/oder unterschiedliche Eigenschaften aufweisen, wobei wobei jeder Wandler (3) des Wandlersatzes (3) an einem Halteelement befestigt ist, das die gleiche Größe und die gleichen Eigenschaften wie das zweite Halteelement (110) aufweist, so dass jeder Wandler (3) der Vielzahl von Wandlern (3) so konfiguriert ist, dass er in der photoakustischen Detektionsvorrichtung (1) austauschbar ist.

## Revendications

1. Dispositif de détection photoacoustique (1) pour mesurer un paramètre d'intérêt dans un milieu comprenant:
- Un boîtier (7) comprenant une plaque de base (72) et un couvercle (71),
- Une source lumineuse (2), configurée pour émettre un signal lumineux
- une cellule photoacoustique (9) comprenant une surface de contact creuse (91), émergeant d'une ouverture (721) de la plaque de base (72), ladite surface de contact creuse (91) étant destinée à être en contact avec le milieu, dans laquelle le signal lumineux est capable de se propager dans la cellule photoacoustique (9) et de traverser la surface de contact creuse (91) pour atteindre le milieu,
- un élément de guidage (8), configuré pour diriger le signal lumineux vers la cellule photoacoustique (9),
- un transducteur (3) relié à la cellule photoacoustique (9) et configuré pour détecter un signal généré, le signal généré étant généré dans la cellule photoacoustique (9) par un effet photothermique dans le milieu en réponse à une irradiation du milieu par le signal lumineux,
- Un premier élément de maintien (100) configuré pour assembler la source lumineuse (2) et l'élément de guidage (8) dans un bloc de construction, ledit bloc de construction étant logé dans le boîtier (7)
**Caractérisé en ce que**
∘ la cellule photoacoustique (9) est fixée de manière réversible au bloc de construction par au moins un élément de fixation (95) accessible depuis l'extérieur du boîtier (7), **en ce qu'**une taille de la cellule photoacoustique (9) est telle que la cellule photoacoustique (9) peut être retirée du boîtier (7) en passant par l'ouverture (721) de la plaque de base (72) si nécessaire, et **en ce que**
∘ le transducteur (3) est couplé de manière réversible au bloc de construction et placé entre ledit bloc de construction et ladite cellule photoacoustique (9), de sorte que le transducteur (3) peut être retiré de l'ouverture (721) de la plaque de base (72) en même temps ou après que la cellule photoacoustique (9) a été ou est retirée du boîtier (7).

2. Dispositif de détection photoacoustique (1) selon la revendication 1, dans lequel le transducteur (3) est fixé à un deuxième élément de maintien (110), le deuxième élément de maintien (110) comprenant une partie de connexion (111) par rapport au premier élément de maintien (100), et dans lequel un élément de connexion est configuré pour connecter de manière réversible ladite partie de connexion (111) dudit deuxième élément de maintien (110) au premier élément de maintien (100) de manière à assurer une connexion mécanique, électronique et/ou électrique entre le transducteur (3) et le bloc fonctionnel.

3. Dispositif de détection photoacoustique (1) selon la revendication 2, dans lequel le retrait du transducteur (3) à l'extérieur du boîtier (7) comprend le retrait du transducteur (3) et du deuxième élément de maintien (110).

4. Dispositif de détection photoacoustique (1) selon les revendications 2 ou 3, dans lequel l'élément de connexion est brasé sur le premier élément de maintien (100).

5. Dispositif de détection photoacoustique (1) selon l'une quelconque des revendications 2 à 4, dans lequel l'élément de connexion est un connecteur à ressort à lame (31).

6. Dispositif de détection photoacoustique (1) selon l'une quelconque des revendications 2 à 5, dans lequel la fixation de la cellule photoacoustique (9) au dispositif de détection (1) avec l'élément de fixation (95) est configurée pour presser le deuxième élément de maintien (110) contre l'élément de connexion.

7. Dispositif de détection photoacoustique (1) selon l'une quelconque des revendications 2 à 6, dans lequel la cellule photoacoustique (9) comprend une partie extensible (97) fixée de manière réversible au deuxième élément de maintien (110), ladite partie extensible (97) entourant le transducteur (3).

8. Dispositif de détection photoacoustique (1) selon la revendication 7, dans lequel la partie extensible (97) de la cellule photoacoustique (9) comprend un goujon (97) configuré pour coopérer avec un trou (112) prévu sur le deuxième élément de maintien (110).

9. Dispositif de détection photoacoustique (1) selon l'une quelconque des revendications 2 à 6, dans lequel la cellule photoacoustique (9) comprend une partie extensible (97) fixée de manière permanente au deuxième élément de maintien (110), ladite partie extensible entourant le transducteur (3).

10. Dispositif de détection photoacoustique (1) selon les revendications 2 à 9, dans lequel la cellule photoacoustique (9) est fixée au deuxième élément de maintien (110) par un adhésif, tel qu'un ruban adhésif double face.

11. Dispositif de détection photoacoustique (1) selon l'une quelconque des revendications 1 à 10, dans lequel l'élément de fixation (95) fixant de manière réversible la cellule photoacoustique (9) au bloc de construction sont des éléments de vissage, dans lequel les têtes des éléments de vissage sont accessibles depuis l'extérieur du boîtier (7).

12. Dispositif de détection photoacoustique (1) selon l'une quelconque des revendications 1 à 11, comprenant en outre une jupe (96) prévue sur la surface de la surface de contact creuse (91) de la cellule photoacoustique (9), ladite jupe (96) étant fixée de manière réversible au dispositif de détection (1).

13. Kit de dispositif de détection photoacoustique comprenant :
- un dispositif de détection photoacoustique (1) selon l'une quelconque des revendications 1 à 12,
- une pluralité de cellules photoacoustiques (9) présentant des propriétés acoustiques différentes et ayant le même élément de fixation réversible (95) de sorte que chaque cellule photoacoustique (9) de la pluralité de cellules photoacoustiques (9) est configurée pour être interchangeable dans le dispositif de détection photoacoustique (1).

14. Kit de dispositif de détection photoacoustique selon la revendication 13, comprenant en outre une pluralité de transducteurs (3) étant de types différents et/ou présentant des propriétés différentes, chaque transducteur (3) du kit de transducteurs (3) étant fixé à un élément de maintien ayant la même taille et les mêmes caractéristiques que le deuxième élément de maintien (110) de telle sorte que chaque transducteur (3) de la pluralité de transducteurs (3) soit configuré pour être interchangeable dans le dispositif de détection photoacoustique (1).
